# EUROPEAN PATENT APPLICATION

(11) **EP 1 699 232 A2**
(43) Date of publication of application: **06.09.2006**
(21) Application number: 06251143.1
(22) Date of filing: 02.03.2006
(51) Int. Cl.: H04N 5/32

(54) **Non-planar x-ray image sensor**

(30) Priority: 03.03.2005 GB 0504415
(71) Applicant: E2V Technologies (UK) Limited, Chelmsford, Essex CM1 2QU (GB)
(72) Inventor: Wood, Scott Alan, Chelmsford Essex CM1 6EA (GB)
(74) Representative: Want, Clifford James

(57) **Abstract**

A non-planar x-ray sensor 40 has a scintillator-coated, thinned photoelectric sensor array on a flexible semiconductor die 45 conformed and affixed to a non-planar printed circuit board 43 providing electrical connections to the photoelectric sensor array. The sensor has particular application as an intra-oral cavity sensor for dental x-ray digital imaging.

## Description

This invention relates to a non-planar x-ray sensor having a semiconductor photoelectric sensor array conformed to a non-planar printed circuit board. The invention has particular application to dental intra-oral radiography.

Charge coupled device (ccd) x-ray imaging sensors with, for example, 912 x 1368 pixels and an image area of 20 mm x 30 mm, are known for digital intra-oral dental applications, and permit a lower x-ray dose than photographic systems. They also produce an image without the delay necessary for photographic development, avoid the storage and use of photographic developing chemicals and facilitate digital archiving of images.

Known sensors include an inflexible, sealed, planar, packaged, ccd array for insertion in a mouth to be examined, the package being typically 25 mm x 39.5 mm x 5.7 mm excluding a cable connection. In use the sensor is typically accommodated in a holder attached to an x-ray source to maintain the sensor at a predetermined distance from, and at a predetermined orientation to, the x-ray source. However, the inflexible, planar nature of the sensor may prevent comfortable accommodation of the sensor within a mouth and limits a vertical extent of the oral cavity which may be radiographed. In particular, it is uncomfortable to close a jaw, as may be required for radiography, with the sensor inside the mouth.

It is desirable to produce a ccd sensor which conforms at least partly to a curvature of the oral cavity to at least one of: facilitate location within the oral cavity, to provide greater patient comfort and to increase a vertical extent of the oral cavity of an image obtained.

US-2003/0187349-A1 discloses an x-ray camera unit for insertion on an endoscope into a body cavity, the camera including a convex curved ccd sheet disposed along an inner face of convex combined, or separate, scintillator and collimator layers, for detecting radioactive tracers. There is no disclosure of the nature of the curved ccd sheet or its manufacture, or the reason for the convex curvature of the ccd sheet other than to conform to the convex collimator layer which defmes an angle of view. The camera unit itself is not curved for accommodation in the body cavity. There is also disclosed an arc-shaped scanning linear array x-ray detector comprising multiple x-ray detector units, disposed along an arcuate face of an endoscope, which are sequentially switched and, in another embodiment, multiple x-ray detector elements wrapped around a cylindrical face of an endoscope. However, there is no disclosure that the x-ray detector units are themselves flexible or non-planar.

US-5547455-B, US-5880341-B and US-5800341-B disclose an endoscope with a substantially semi-spherical convex array of light-receiving ccd cells and a circumferential band of ccd cells around a distal end of the endoscope shaft. There is no disclosure that individual cells are flexible or non-planar.

US-5134680-B discloses a ccd x-ray camera having a 1 cm square or 2 cm square (1024 x 1024 pixels) ccd die with what is described as a disadvantageous fixed radius of curvature of 4.7 to 6.1 metres, which is flattened with a plano-concave fibre optic channel plate.

WO-00/55866-A discloses a back-illuminated ccd camera for dental applications in which the ccd array may have a curved layout for use with a gamma-ray source with the source inside a mouth and the camera outside. By providing a cyclic motion of the array a slice-by-slice image of an object may be captured. There is no disclosure that the ccd array is flexible, in which sense the array is curved, or the purpose of the curvature and, in particular, there is no disclosure of a non-planar x-ray sensor for insertion in an oral cavity.

WO-94/04001-A discloses a panoramic camera with a concave ccd sensor designed in a radial form centred on an objective lens, presumably to conform to a focal field of the lens.

US-5880777-B, US-5909244-B, US-2001/0019361-A and US-6489992-B2 disclose a low-light level visible or infrared wavelength camera having a thinned, flexible ccd imager substrate deformed to adhere by epoxy to a concave, spherically-curved support substrate, such that an image plane of a single-element simple lens is in focus over a wide field of view. A complex vacuum forming process is described for forming the ccd imager substrate to a support substrate having a plurality of concave indentations before dicing the support substrate and the overlying ccd imager substrate into individual ccd imager dies.

US2004/0238750 appears to disclose an X-ray detector comprising a curved glass layer, a photoreceptor formed on the glass layer and a curved backing layer having a same radius of curvature as the glass layer to support the glass layer, to overcome imaging distortions caused by a flat or adversely curved (i.e. convex towards the source) imagers in which portions of the imager are at different distances from, and angles to, an X-ray source. The detector is therefore concave towards the source. The backing layer includes routing connection points, for example bump pads, flat pads, pins and receptacles, with vias in the backing layer carrying signals from the photoreceptor layer. The backing layer may be formed from stacked, multiple layers using a process suitable for constructing multiple layer printed circuit boards. The thin glass layer is thin enough to bend along the radius of curvature without breaking. In manufacture a thin glass layer is bonded to a polymer support layer, electronic layers are deposited on the glass layer, e.g. by CVD, to form photodiodes or FETs. A ceramic backing structure, including metallized traces, is fabricated and formed into a curved shape and fired to a rigid form. The flexible glass and polymer structure is curved to the desired geometry and bonded to the backing layer.

It is an object of the present invention at least to ameliorate the aforesaid shortcomings in the prior art.

According to a first aspect of the present invention there is provided a non-planar x-ray sensor comprising semiconductor photoelectric sensor array means conformed and affixed to non-planar printed circuit board means providing electrical connectivity to the semiconductor photoelectric sensor array means, wherein the semiconductor photoelectric sensor array means is thinned after manufacture thereof to render the semiconductor photoelectric sensor array means sufficiently flexible to conform to a surface of the non-planar printed circuit board means.

Conveniently, the non-planar printed circuit board means is heat-formable printed circuit board means.

Conveniently, the semiconductor photoelectric sensor array means has a thickness of substantially 60µ.

Preferably, the photoelectric sensor array means comprises charge coupled devices.

Conveniently, the photoelectric sensor array means comprises CMOS devices.

Conveniently, the photoelectric sensor array means is affixed to the printed circuit board means with adhesive.

Conveniently, the photoelectric sensor array means is affixed to the printed circuit board means with double-sided adhesive tape

Advantageously, the photoelectric sensor array means is coated with scintillator or phosphor means for emitting, on irradiation by x-rays, light of a wavelength suitable for stimulating the photoelectric sensor array means.

Preferably, the photoelectric sensor array means is electrically connected to the non-planar printed circuit board means by wire bonding means.

Advantageously, the non-planar x-ray sensor comprises non-planar packaging means for insertion into an oral cavity for dental digital radiography.

Preferably, the non-planar packaging means conforms substantially to a non-planar surface of the oral cavity.

Conveniently, the non-planar x-ray sensor is substantially rectangular such that a major axis of the sensor may be accommodated substantially in an opening direction of an oral cavity.

Conveniently, the non-planar x-ray sensor comprises electrical cable means electrically connected to the printed circuit board means at least for extracting signals from the semiconductor photoelectric sensor array means.

Preferably, the electrical cable means is aligned with respect to the x-ray sensor such that, in use, the electrical cable means may be accommodated substantially perpendicular to an opening direction of an oral cavity.

Advantageously, the non-planar x-ray sensor is a dental intra-oral cavity x-ray sensor.

According to a second aspect of the invention, there is provided a method of manufacturing a non-planar x-ray sensor comprising the steps of: providing non-planar printed circuit board means; providing semiconductor photoelectric sensor array means sufficiently flexible to conform to a surface of the non-planar printed circuit board means by thinning the photoelectric sensor array means after manufacture thereof; conforming and affixing the semiconductor photoelectric sensor array means to the surface; and making electrical connections between the semiconductor photoelectric sensor array means and the printed circuit board means.

Conveniently, the step of providing non-planar circuit board means comprises the steps of: providing substantially inflexible, heat-formable planar printed circuit board means; and forming the heat-formable planar printed circuit board means into a predetermined non-planar shape.

Advantageously, the step of providing photoelectric sensor array means comprises providing an array including at least one of charge coupled devices and CMOS devices.

Advantageously, the step of thinning comprises grinding and polishing a rear face, opposed to a face containing active devices, of the photoelectric sensor array means.

Conveniently, the step of thinning is to a thickness of substantially 60µ.

Preferably, the method comprises a further step of coating the photoelectric sensor array means with scintillator or phosphor means for emitting, on irradiation by x-rays, light of a wavelength suitable for stimulating the photoelectric sensor array means.

Conveniently, the step of coating comprises one of spin-coating, vapour depositing or screen-printing a coating.

Preferably, the step of conforming and affixing the photoelectric sensor array means comprises affixing the photoelectric sensor array means to the printed circuit board means with adhesive.

Alternatively, the step of conforming and affixing the photoelectric sensor array means comprises affixing the photoelectric sensor array means to the printed circuit board means with double-sided adhesive tape.

Preferably, the method comprises a further step of packaging the photoelectric sensor array means affixed to the printed circuit board means in non-planar packaging means.

Preferably, the non-planar packaging means conforms at least partially to a non-planar surface of an intra-oral cavity for dental radiography.

Advantageously, the non-planar x-ray sensor is a dental intra-oral cavity x-ray sensor.

The invention will now be described, by way of example, with reference to the accompanying drawings in which:
Figure 1 is a perspective view from a rear, top or bottom and one side of a prior art dental x-ray sensor;
Figure 2 is a schematic rear view of the prior art sensor of Figure 1;
Figure 3 is a vertical cross-section along line 3-3 of Figure 2;
Figure 3A is an enlarged representation of the circled portion of Figure 3;
Figure 4 is a schematic rear view of a sensor according to a first aspect of the invention;
Figure 5 is a vertical cross-section along line 5-5 of Figure 4;
Figure 5A is an enlarged representation of the circled portion of Figure 5;
Figure 6 is a flowchart of a method of manufacture, according to a second aspect of the invention, of the sensor of Figure 4;
Figure 7 is a schematic side view of the sensor of Figure 4 in use in a first position with respect to a tooth to be imaged; and
Figure 8 is a schematic side view of the sensor of Figure 4 in use in a second position with respect to the tooth to be imaged.

In the Figures like reference numerals denote like parts.

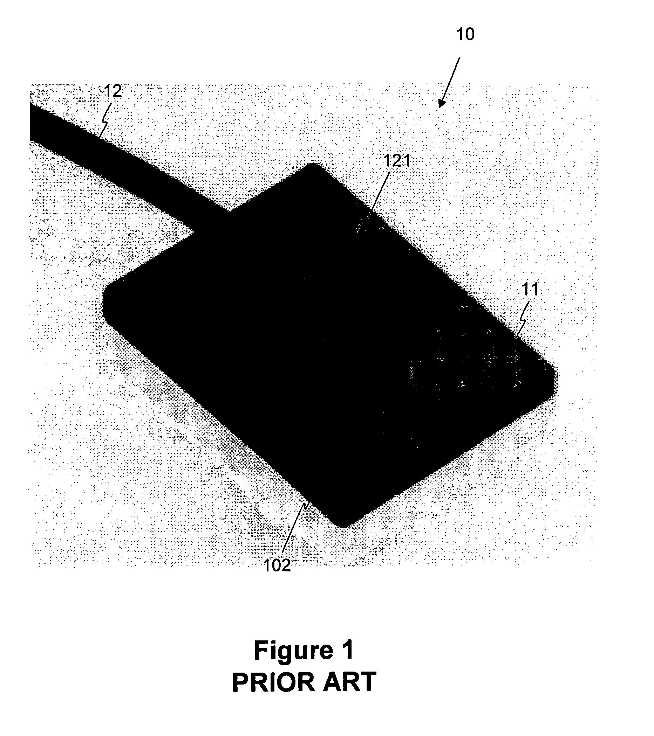

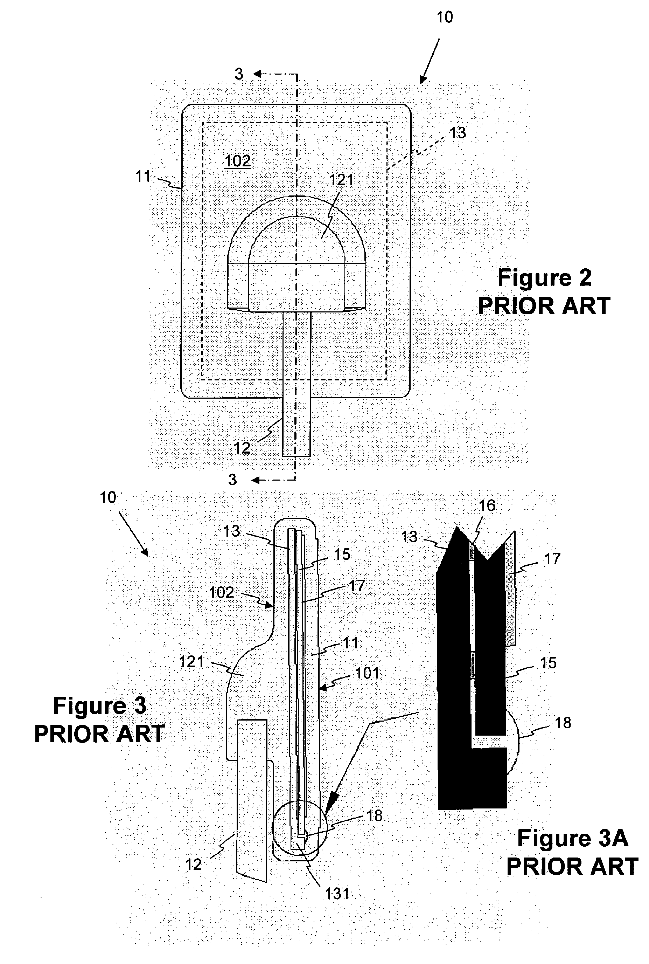

Referring to Figures 1 to 3A, a prior art dental x-ray sensor 10 is encased in an inflexible, sealed, substantially rectangular, planar case 11. An electrical cable 12 is connected substantially centrally of a back face 102 of the case 11 by a cable blister 121, such that the cable 12 is substantially aligned with a major axis of the substantially rectangular case 11. As shown best in Figures 2 and 3, within the case 11 there is provided a planar, substantially rectangular printed circuit board 13, fixed substantially central of the case 11 by abutting inner surfaces of walls thereof. A lower portion, as shown in Figure 3, of the printed circuit board 13 is provided with a return portion 131, forward of the plane of the printed circuit board and away from the back face, bearing electrical connectors, not shown, on a front face thereof such that in vertical cross-section, as shown in Figure 3, the printed circuit board is L-shaped. A semiconductor die 15 comprising an array of charged coupled devices is adhered by a rear face thereof to, and spaced from, the printed circuit board 13 by adhesive deposits 16, as shown in Figure 3A. A front face of the semiconductor die, opposed to the rear face, is substantially coplanar with a front face of the return portion 131 of the printed circuit board 13. Apart from a portion of the front face of semiconductor die 15 containing electrical connectors, not shown, proximate the return portion 131 of the printed circuit board 13, substantially all of the front face of the semiconductor die 15 is coated with a scintillator, or phosphor, layer 17. Electrical connectors on the semiconductor die 15 are connected to electrical connectors on the printed circuit board 13 by wire bonds 18. The electrical connectors on the printed circuit board are connected by tracking thereof, not shown, to terminations of the cable 12. In use the cable 12 and a major axis of the sensor are substantially perpendicular to an opening direction of a mouth to be imaged.

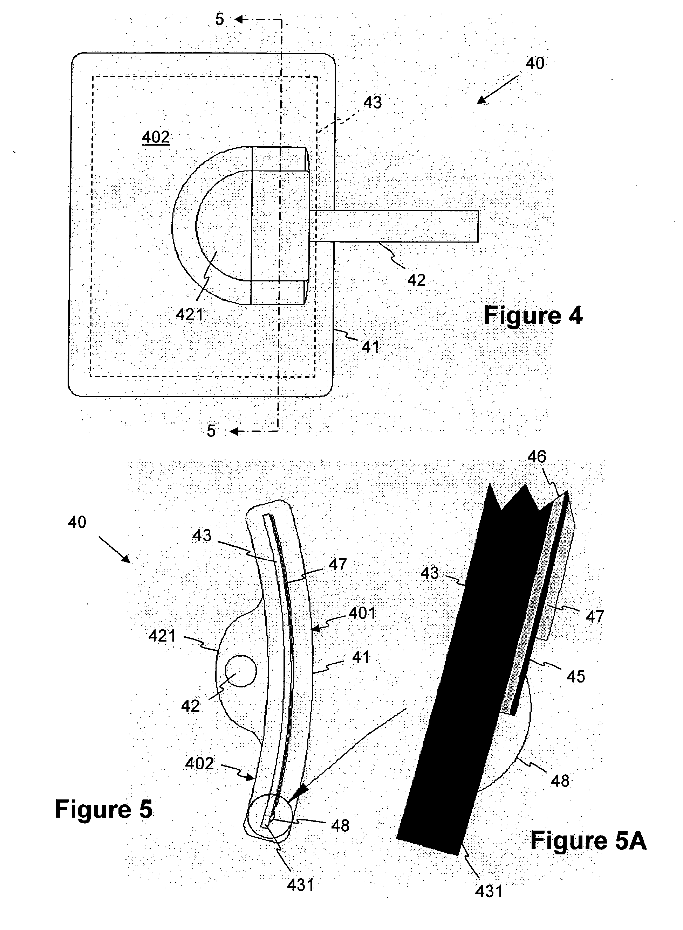

Referring to Figures 4 to 5A a dental x-ray sensor 40 according to the invention is encased in a substantially inflexible, sealed, substantially rectangular, housing or case 41, arcuate in cross-section parallel to a major axis thereof. An electrical cable 42 is connected substantially centrally of a back face of the arcuate case 41 by a cable blister 421, such that the cable 42 is aligned with a minor axis of the substantially rectangular, arcuate case 41. As best seen in Figures 4 and 5, within the arcuate case 41 there is provided a substantially rectangular printed circuit board 43, arcuate in cross-section parallel to a major axis thereof, fixed substantially centrally of the arcuate case 42 by abutting inner surfaces of walls thereof. A lower portion, as shown in Figure 5, of the arcuate printed circuit board bears electrical connectors, not shown, on a front face thereof. A semiconductor die 45, thin in comparison with the prior art semiconductor die 15, comprising an array of charged coupled devices is adhered by a rear face thereof to, and spaced from, the printed circuit board 43 by a layer of adhesive 46. Alternatively, the thinned semiconductor die may be fixed to the arcuate printed circuit board 43 by double-sided adhesive tape. Apart from a portion of the front face of thinned semiconductor die 45 containing electrical connectors, not shown, proximate a lower portion 431, as orientated in Figure 5, of the printed circuit board 43, substantially all of the front face of the thinned semiconductor die 45 is coated with a scintillator, or phosphor, layer 47 for emitting, on irradiation by x-rays, light of a wavelength suitable for stimulating the charge coupled device array of the semiconductor die 45. Electrical connectors on the semiconductor die 45 are connected to electrical connectors on the lower portion 431 of the printed circuit board 43 by wire bonds 48. It will be understood that other known methods of making electrical connection between the semiconductor die and the printed circuit board, such as solder bumps, may be used. The electrical connectors on the printed circuit board are connected by tracking thereof, not shown, to terminations of the cable 42.

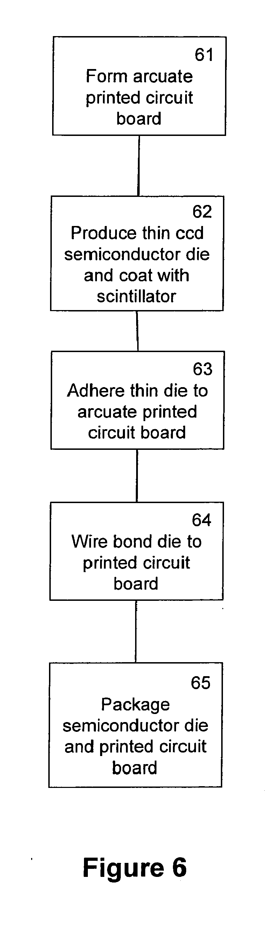

Referring in particular to Figure 6, the sensor 40 may be manufactured as follows. A heat-deformable planar printed circuit board is heated and formed, step 61, such that the board is arcuate parallel to a major axis thereof, with an outer radius of substantially 6 cm and allowed to cool, retaining an arcuate shape, to form the arcuate printed circuit board 43. However, it will be understood that the printed circuit board may alternatively be formed into some other non-planar shape, such as a dome, into which the semiconductor array can conform, for other dental or non-dental applications, or formed in a non-planar shape in some other known manner. Utilising a known mechanical grinding and polishing process, a semiconductor die containing an array of ccd devices is back-thinned, step 62, to a total thickness of substantially 60µ to form the thinned semiconductor die 45. It will be understood that other known means of thinning semiconductor wafers, such as chemical etching or laser ablation may be used. At this thickness, although fragile, the silicon die is sufficiently flexible for mounting on a non-planar substrate. The thinned die is coated, for example by spin-coating, with a phosphor 47 such as red-emitting gadolinium oxysulphide. Alternatively the phosphor coating is screen-printed or evaporated onto the planar thinned die. The back-thinned, coated, semiconductor die 45 containing the ccd array is affixed to a convex surface of the arcuate printed circuit board 43 with adhesive 46 or double-sided adhesive tape, with a front light-sensitive face of the ccd array outermost. Alternatively, an uncoated, thinned, semiconductor die is affixed to the arcuate printed circuit and the phosphor coating is screen-printed or evaporated onto the arcuate assembly of thinned die and arcuate printed circuit board. Electrical contacts on the semiconductor die 45 are wire bonded, step 64, to contacts on the arcuate printed circuit board 43 for electrical connection through tracking on the circuit board to the electrical cable 42. When the ccd device array on the semiconductor die 45 is powered through the electrical cable 42 the ccd array captures x-ray images whilst fixed in the arcuate position on the arcuate printed circuit board 43. The assembled arcuate ccd array semiconductor die 45 and arcuate printed circuit board 43 may be packaged, step 65, in an arcuate package 41, substantially transparent to x-rays, at least on a front face 401 thereof, to form a curved inter-oral dental ccd imaging device 40, suitable for comfortable accommodation in an oral cavity. Although the use of a semiconductor die illuminated by x-rays from a front surface containing the ccd array has been described, it will be understood that a back-illuminated semiconductor die, in which the active devices are illuminated through an x-ray transparent substrate of the die, may be used.

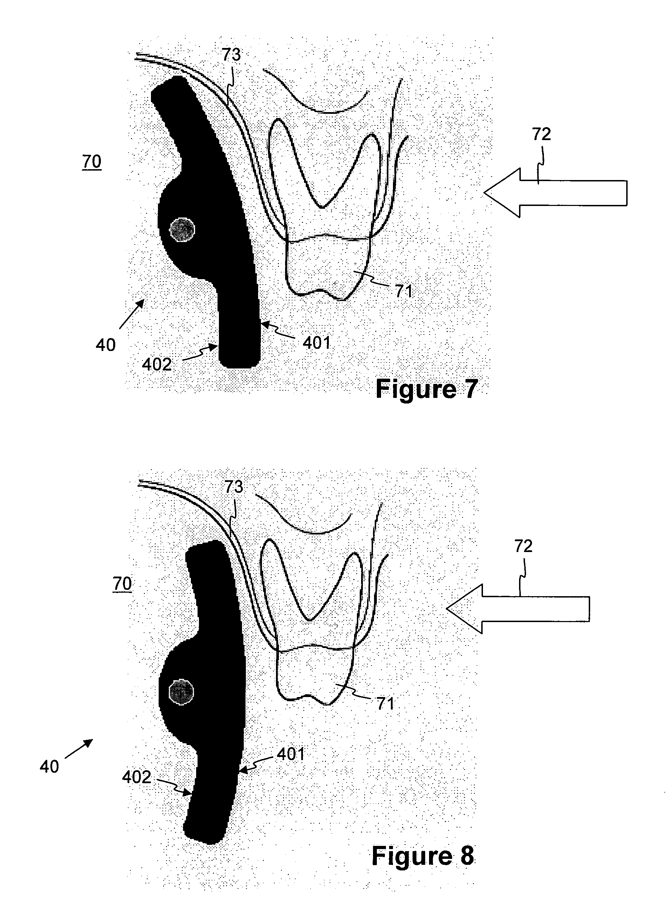

Referring to Figures 7 and 8, in use the imaging device is inserted in an oral cavity 70 with a front face 401 of the sensor towards a tooth 71 to be imaged. X-rays from an x-ray source, not shown, are incident on the tooth 71 from outside the oral cavity, substantially in a direction of arrow 72 in Figures 7 and 8, to form an image of the tooth 71 on the phosphor 47. Resultant luminescence of the phosphor or scintillator 47 excites the ccd array in a known manner for the formation of a digital image by processing signals from the sensor. As can be seen in Figures 7 and 8, in either a lower or higher position within the oral cavity, curvature of the x-ray sensor 40 allows the sensor to fit more comfortably in a patient's mouth than prior art planar sensors, whilst still imaging a required area of jaw. Moreover, the curvature of the sensor, and a cable substantially parallel with a minor axis of the sensor, rather than with a major axis as in the prior art, allows a sensor with imaging dimensions substantially the same as a prior art sensor to be used with the major axis substantially vertical, that is in an opening direction of the oral cavity, rather than substantially horizontal with respect to the oral cavity, that is substantially perpendicular to the opening direction of the oral cavity, as in the prior art, allowing a greater vertical extent of the oral cavity to be imaged simultaneously.

Surprisingly, images obtained with the curved dental sensor are virtually indistinguishable from those obtained with planar sensors. However, if necessary, known field flattening signal or data processing is applied to the resultant images to correct for any distortion caused by the non-planar nature of the ccd array.

It will be understood that the method of the invention has the advantage of producing non-planar ccd arrays without a requirement for non-planar semiconductor lithography.

Although the x-ray sensor has been described in relation to a dental intra-oral sensor, a person skilled in the art will appreciate that the invention has application wherever a non-planar photoelectric sensor is required, for example for endoscopes or industrial, astronomical or space applications.

## Claims

1. A non-planar x-ray sensor (40) **characterised by** semiconductor photoelectric sensor array means (45) conformed and affixed to non-planar printed circuit board means (43) providing electrical connectivity to the semiconductor photoelectric sensor array means, wherein the semiconductor photoelectric sensor array means (45) is thinned after manufacture thereof to render the semiconductor photoelectric sensor array means sufficiently flexible to conform to a surface of the non-planar printed circuit board means.

2. A non-planar x-ray sensor as claimed in claim 1, wherein the non-planar printed circuit board means (43) is heat-formable printed circuit board means.

3. A non-planar x-ray sensor as claimed in claims 1 or 2, wherein the semiconductor photoelectric sensor array means (45) has a thickness of substantially 60µ.

4. A non planar x-ray sensor as claimed in any of the preceding claims, wherein the photoelectric sensor array means comprises charge coupled devices.

5. A non-planar x-ray sensor as claimed in any of the preceding claims, wherein the photoelectric sensor array means comprises CMOS devices.

6. A non-planar x-ray sensor as claimed in any of the preceding claims, wherein the photoelectric sensor array means is affixed to the printed circuit board means with adhesive (46).

7. A non-planar x-ray sensor as claimed in any of the preceding claims, wherein the photoelectric sensor array means is affixed to the printed circuit board means with double-sided adhesive tape.

8. A non-planar x-ray sensor as claimed in any of the preceding claims, wherein the photoelectric sensor array means is coated with scintillator or phosphor means (47) for emitting, on irradiation by x-rays, light of a wavelength suitable for stimulating the photoelectric sensor array means.

9. A non-planar x-ray sensor as claimed in any of the preceding claims, wherein the photoelectric sensor array means is electrically connected to the non-planar printed circuit board means by wire bonding means (48).

10. A non-planar x-ray sensor as claimed in any of the preceding claims, comprising non-planar packaging means for insertion into an oral cavity (70) for dental digital radiography.

11. A non-planar x-ray sensor as claimed in claim 10, wherein the non-planar packaging means conforms substantially to a non-planar surface of the oral cavity.

12. A non-planar x-ray sensor as claimed in any of the preceding claims, wherein the sensor is substantially rectangular such that a major axis of the sensor may be accommodated substantially in an opening direction of an oral cavity (70).

13. A non-planar x-ray sensor as claimed in any of the preceding claims, comprising electrical cable means (42) electrically connected to the printed circuit board means at least for extracting signals from the semiconductor photoelectric sensor array means.

14. A non-planar x-ray sensor as claimed in claim 13, wherein the electrical cable means is aligned with respect to the x-ray sensor such that, in use, the electrical cable means may be accommodated substantially perpendicular to an opening direction of an oral cavity.

15. A non-planar x-ray sensor as claimed in any of the preceding claims, wherein the non-planar x-ray sensor is a dental intra-oral cavity x-ray sensor.

16. A method of manufacturing a non-planar x-ray sensor comprising the steps of:
a) providing non-planar printed circuit board means (43);
b) providing (62) semiconductor photoelectric sensor array means (45) sufficiently flexible to conform to a surface of the non-planar printed circuit board means by thinning the photoelectric sensor array means after manufacture thereof;
c) conforming and affixing (63) the semiconductor photoelectric sensor array means to the surface; and
d) making (64) electrical connections (48) between the semiconductor photoelectric sensor array means and the printed circuit board means.

17. A method as claimed in claim 16, wherein the step of providing non-planar circuit board means comprises the steps of: providing substantially inflexible, heat-formable planar printed circuit board means; and forming (61) the heat-formable planar printed circuit board means into a predetermined non-planar shape.

18. A method as claimed in claims 16 or 17, wherein the step of providing semiconductor photoelectric sensor array means comprises providing an array including at least one of charge coupled devices and CMOS devices.

19. A method as claimed in any of claims 16 to 18, wherein the step of thinning comprises grinding and polishing a rear face, opposed to a face containing active devices, of the photoelectric sensor array means.

20. A method as claimed in any of claims 16 to 19, wherein the step of thinning is to a thickness of substantially 60µ.

21. A method as claimed in any of claims 16 to 20, comprising a further step of coating the photoelectric sensor array means (45) with scintillator or phosphor means (47) for emitting, on irradiation by x-rays, light of a wavelength suitable for stimulating the photoelectric sensor array means.

22. A method as claimed in claim 21, wherein the step of coating comprises one of spin-coating, vapour depositing or screen-printing a coating.

23. A method as claimed in any of claims 16 to 22, wherein the step of conforming and affixing the photoelectric sensor array means comprises affixing the photoelectric sensor array means to the printed circuit board means with adhesive (46).

24. A method as claimed in any of claims 16 to 22, wherein the step of conforming and affixing the photoelectric sensor array means comprises affixing the photoelectric sensor array means to the printed circuit board means with double-sided adhesive tape.

25. A method as claimed in any of claims 16 to 24, comprising a further step of packaging (65) the photoelectric sensor array means affixed to the printed circuit board means in non-planar packaging means.

26. A method as claimed in claim 25, wherein the non-planar packaging means conforms at least partially to a non-planar surface of an intra-oral cavity (70) for dental radiography.

27. A method as claimed in any of claims 16 to 26, wherein the non-planar x-ray sensor is a dental intra-oral cavity x-ray sensor.
